# EUROPEAN PATENT APPLICATION

(11) **EP 0 566 882 A1**
(43) Date of publication of application: **27.10.1993**
(21) Application number: 93104732.8
(22) Date of filing: 23.03.1993
(51) Int. Cl.: A61M 5/32, A61M 5/50

(54) **Safety syringe**

(30) Priority: 25.03.1992 IT MI920710
(71) Applicant: Federico, Demetrio, I-20032 Cormano (Milano) (IT)
(72) Inventor: Federico, Demetrio, I-20032 Cormano (Milano) (IT)
(74) Representative: Modiano, Guido, Dr.-Ing.

(57) **Abstract**

The safety syringe for preventing its reuse and accidental punctures with the needle has a cylindrical body (2) in which a plunger-like piston (11) is tightly slideable; the piston defines, in the cylindrical body, a chamber for containing a liquid to be injected; a needle (15) is removably connected to the cylindrical body. The syringe also has helical protrusions (20,22,32,33) for the removable connection of the needle which can be actuated by the plunger-like piston at the end of its injection stroke and to disengage the needle from the cylindrical body. There are also springs (40) which can be activated at the end of the injection stroke of the plunger-like piston to move the plunger-like piston in the direction opposite to the injection stroke and to retract the needle into the chamber.

## Description

The present invention relates to a safety syringe with means for preventing its reuse and accidental punctures with the needle.

As is known, so-called single-use disposable syringes are currently in ever-increasing use; generally, they are constituted by a body made of plastics inside which a plunger-like piston for injecting the liquid is slidably accommodated.

A needle, also of the single-use type, is applied by pressure to the syringe body.

This embodiment allows excellent sterilization up to the time of use, but these syringes must not be reused, since they cannot be resterilized.

The adoption of single-use syringes has currently brought about the severe danger of the spread of contagion of dangerous and incurable diseases, both due to accidental punctures with syringes discarded into the environment and to the reuse of used syringes.

Currently, no satisfactory solutions have been found to prevent reuse of syringes and to prevent the used needle from being a potential hazard for accidental punctures.

Solutions which, in order to prevent reuse of the syringes, cause, after first use, the automatic retraction of the needle inside the syringe body, thus preventing both reuse and the possibility of accidental punctures, have already been provided for; these solutions are disclosed for example in Italian patents nos. 1,227,228 and 1,227,272 in the name of the same Applicant.

These solutions are undoubtedly valid from a theoretical point of view, but they have the drawback that they require, for their correct use, a succession of movements which differ from those conventionally performed when a syringe is used.

This fact, besides creating considerable constructive complications, is certainly a severe limitation to the possibilities of widespread diffusion of this syringe among users.

With these syringes it is in fact necessary to apply on the plunger-like piston, in addition to the conventional pressure required to perform the injection stroke, an additional pressure to load the spring which would then produce the return stroke of the piston after it has engaged the needle.

The user would furthermore have to maintain his grip on the plunger-like piston throughout the period during which he positions the needle after eliminating the air drawn-in together with the liquid during the introduction of the liquid in the syringe.

This hindrance would certainly constitute a severe obstacle to the large-scale diffusion of syringes manufactured with this criterion.

The aim of the present invention is to solve the problems described above by providing a safety syringe with means for preventing its reuse and accidental punctures with the needle, which allows to use the syringe like a conventional one but with the advantage that, once injection has been performed, the needle is automatically retracted inside the syringe body.

Within the scope of the above aim, a particular object of the present invention is to provide a syringe which, despite having considerably improved characteristics from the point of view both of safety and practicality, has a relatively simple structure.

Another object of the present invention is to provide a safety syringe which, by virtue of its particular constructive characteristics, is capable of giving the greatest assurances of reliability and safety in use.

Not least object of the present invention is to provide a safety syringe which can be easily obtained starting from commonly commercially available elements and materials and is furthermore competitive from a merely economical point of view.

This aim, these objects and others which will become apparent hereinafter are achieved by a safety syringe with means for preventing its reuse and accidental punctures with the needle, according to the invention, comprising a cylindrical body in which a plunger-like piston is tightly slideable, said piston defining, in said cylindrical body, a chamber for containing a liquid to be injected, a needle being removably connected to said cylindrical body; characterized in that it comprises means for the removable connection of said needle which can be actuated by said plunger-like piston at the end of the injection stroke to disengage said needle from said cylindrical body; elastic means being furthermore provided which can be activated at the end of the injection stroke of said plunger-like piston for the movement of said plunger-like piston in the direction opposite to the injection stroke and for the retraction of said needle into said chamber.

Further characteristics and advantages will become apparent from the description of a preferred but not exclusive embodiment of a safety syringe with means for preventing its reuse and accidental punctures with the needle, illustrated only by way of non-limitative example in the accompanying drawings, wherein:

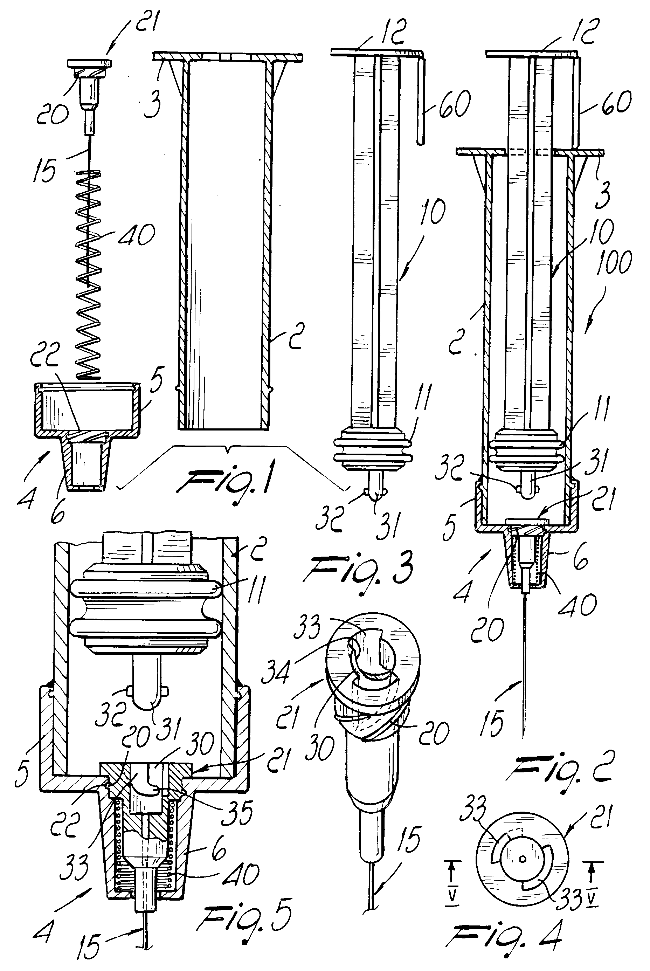

figure 1 is a schematic exploded view of the safety syringe according to the present invention, with elastic means arranged inside the syringe;
figure 2 is a sectional view of the safety syringe, as it looks like during packaging;
figure 3 is an enlarged-scale view of the coupling body of the needle;
figure 4 is a plan view of the needle coupling body;
figure 5 is a sectional view, taken along the plane V-V of figure 4, of the needle applied to the cylindrical body;
figure 6 is a schematic view of the introduction of liquid inside the syringe;
figure 7 is a view of the syringe during injection of the liquid;
figure 8 is a sectional view taken along the plane VIII-VIII of figure 7;
figure 9 is a view of the final step, with the retraction of the needle inside the cylindrical body;
figure 10 is a detail view of the needle and of the piston at the end of the injection stroke;
figure 11 is a sectional detail view of the coupling between the piston-like body and the needle;
figure 12 is a sectional view of elastic means arranged outside the cylindrical body of the syringe during the initial step;
figure 13 is a sectional view of the elastic means of figure 12 when the injection stroke ends;
figure 14 is a schematic sectional view of the final step, with the translatory motion of the plunger-like piston in the direction opposite to the injection stroke.

With reference to the above figures, the safety syringe with means for preventing its reuse and accidental punctures with the needle, according to the present invention, generally designated by the reference numeral 100, comprises a cylindrical body 1 which is formed by means of a tubular portion 2 provided with a grip flange 3 at one end and rigidly connected, at the other end, to a needle coupling inlet, generally designated by the reference numeral 4, comprising a cup-like portion 5 which tapers into a conical tang 6.

A plunger-like piston 10 is tightly slideable inside the cylindrical body 1 and is provided, at one end, with the conventional sealing gaskets 11 which act on the internal wall of the tubular body 2; at its other end, said piston is provided with a button-shaped expansion 12 for the actuation of said piston.

A needle, generally designated by the reference numeral 15, can be connected to the cylindrical body 1.

An important particularity of the present invention is constituted by the fact that the needle 15 is connected to the cylindrical body 1 by virtue of connecting means which are arranged inside the conical tang 6 and are substantially constituted by helical protrusions 20 defined on the coupling body 21 of the needle 15 and engaging complementary protrusions 22 defined inside the conical tang 6.

A thread-like coupling is formed in practice and can be engaged or disengaged by turning in one direction or the other.

A cavity 30 is defined inside the coupling body 21 of the needle 15, and a pin 31 can be inserted therein; said pin 31 is arranged at the end of the plunger-like piston 10 and is provided with diametrical protrusions 32.

Coupling between the plunger-like piston and the needle coupling body is provided so that at the end of the injection stroke, as will become apparent hereinafter, the plunger-like piston disengages the needle from the cylindrical body.

For this purpose, the cavity 30 of the needle body has, complementarily with respect to the diametrical protrusions 32, recesses 33 which extend axially and are delimited by an inclined abutment 34 which, by engaging the protrusions 32, since the piston 10 cannot rotate about its own axis, turns the coupling body and consequently disengages it from the cylindrical body 1.

As shown in figure 5, the inclined abutment inside a recess 33 ends with a notch 35 acting as coupling element for the engagement of the plunger-like piston with the needle body.

Correspondingly, the laterally located recess 33 has a free portion which in practice allows, as will become apparent hereinafter, to tilt the needle during its retraction into the chamber.

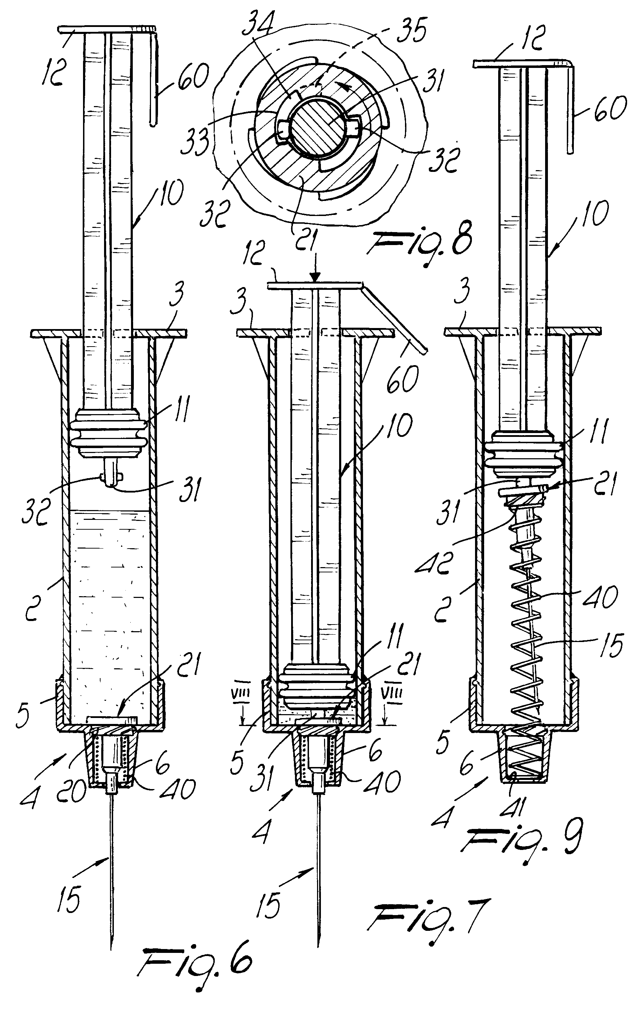

As shown in figures 5 to 9, there are elastic means which can be activated at the end of the injection stroke.

Said elastic means comprise a spring 40 accommodated inside the conical tang 6 and acting between an internal abutment 41 of the tang 6 and a flange 42 defined on the needle coupling body, so that when the needle is turned, disengaging it from the cylindrical body, the needle automatically retracts into the chamber defined in the cylindrical body by the plunger-like piston, with the consequent rise of said piston.

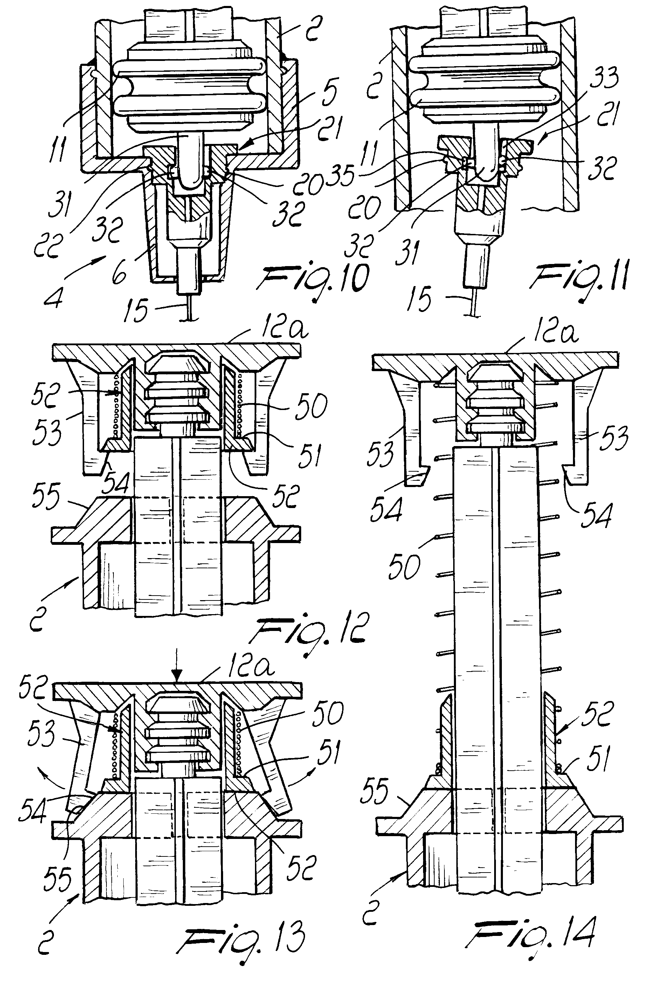

According to a different embodiment illustrated in figures 12 to 14, the elastic means are provided outside the plunger-like body and are accommodated, in compressed position, below the button provided at the end of the plunger-like piston 10, now designated by the reference numeral 12a.

The elastic means comprise a cylindrical spring, designated by the reference numeral 50, which acts between the internal surface of the button 12a and the flanged base 51 of a collar 52 arranged coaxially to the plunger-like piston 10.

The collar 51 is retained in position by tabs 53 ending with an engagement tooth 54 which engages the lower face of the collar 52, keeping the spring in compressed position.

In order to activate the elastic means at the end of the injection stroke, a truncated-cone abutment 55 is provided on the end of the cylindrical body, again designated by the reference numeral 2; at the end of the injection stroke, said abutment 55 opens out the tabs 53, consequently disengaging the teeth 54 from the collar 52 and allowing the expansion of the cylindrical spring 50, with the consequent rise of the piston inside the cylindrical body and retraction of the needle 15 into the chamber formed by said cylindrical body.

To the above it should be added that a removable retaining rod 60 extends from the button 12 and abuts against the flange 3 of the cylindrical body to prevent, during the initial handling of the syringe, the inadvertent coupling of the plunger-like piston to the needle.

In practical use, the syringe is packaged and sterilized in the condition shown in figure 2, in which the needle 15 is coupled to the cylindrical body 2 and the plunger-like piston is partially extracted from the cylindrical body to prevent it from coupling to the needle.

As is customary, in order to fill the syringe the liquid is drawn by moving the plunger-like piston 10 and, as equally customary, the air is then expelled by inverting the syringe.

The syringe, after removing the retainer rod 60 by moving or breaking it off, is then ready for use.

The syringe is used in an absolutely conventional manner, performing the injection stroke of the plunger-like piston 10.

At the end of its stroke, i.e. when all the contained liquid has been delivered, the radial protrusions 32 of the pin 31 provided at the end of the plunger-like piston 10 enter the cavity 30, and the protrusions 32, by engaging the inclined abutment 34, turn the coupling body 21 and consequently disengage the protrusions 20 from the complementary protrusions 22.

The needle is thus free, and the elastic means act; said elastic means can be constituted by the internal spring 40, which pushes the needle inward, or by the external spring 50 which pulls so as to extract the plunger-like piston 10, to which the needle is engaged, from the cylindrical body 2.

As previously mentioned, there is a single notch 35 for the protrusions 32, so that during its insertion in the syringe the needle tilts, so that subsequent reuse of the syringe is not possible since the needle cannot exit.

In these conditions, the needle is accommodated inside the syringe and does not constitute a hazard for accidental punctures; furthermore, it is not possible to reuse the syringe.

From what has been described above it can thus be seen that the syringe according to the invention achieves the intended aim and objects; particularly, the fact is stressed that a syringe is provided which is used in a conventional manner, since the elastic return means are in compressed position during use of the syringe and disengage, applying their return force, only at the end of the injection stroke.

The syringe thus conceived is susceptible to numerous modifications and variations, all of which are within the scope of the inventive concept.

All the details may furthermore be replaced with other technically equivalent elements.

In practice, the materials employed, as well as the contingent shapes and dimensions, may be any according to the requirements.

Where technical features mentioned in any claim are followed by reference signs, those reference signs have been included for the sole purpose of increasing the intelligibility of the claims and accordingly such reference signs do not have any limiting effect on the scope of each element identified by way of example by such reference signs.

## Claims

1. Safety syringe with means for preventing its reuse and accidental punctures with the needle, comprising a cylindrical body in which a plunger-like piston is tightly slideable, said piston defining, in said cylindrical body, a chamber for containing a liquid to be injected, a needle being removably connected to said cylindrical body; characterized in that it comprises means for the removable connection of said needle which can be actuated by said plunger-like piston at the end of the injection stroke to disengage said needle from said cylindrical body; elastic means being furthermore provided which can be activated at the end of the injection stroke of said plunger-like piston for moving said plunger-like piston in the direction opposite to the injection stroke and for retracting said needle into said chamber.

2. Syringe according to claim 1, characterized in that said needle coupling means are arranged inside the conical tang located at the end of said cylindrical body.

3. Syringe according to the preceding claims, characterized in that said coupling means are constituted by helical protrusions defined on the needle coupling body and engaging complementary protrusions defined inside said tang, the engagement and disengagement of said protrusions from said complementary protrusions being obtainable by mutual rotation.

4. Syringe according to one or more of the preceding claims, characterized in that it comprises, inside the coupling body of said needle, a cavity in which a pin is removably insertable, said pin being located at the end of said plunger-like piston and being provided with diametrical protrusions coupleable with the recesses defined in said cavity, said recesses defining an inclined abutment suitable to generate, due to engagement with said protrusions, a rotation of said coupling body with respect to the cylindrical body of the syringe.

5. Syringe according to one or more of the preceding claims, characterized in that one of said inclined abutments ends, at its internal end, with a notch suitable to act as element for coupling to one of said protrusions, for the engagement of said plunger-like piston with said needle.

6. Syringe according to one or more of the preceding claims, characterized in that the recess facing the recess which forms said notch is correspondingly provided with an indent to tilt said needle when it retracts into said chamber.

7. Syringe according to one or more of the preceding claims, characterized in that said elastic means comprise a spring which is accommodated inside said conical tang and acts between an internal abutment of said tang and a flange defined on said needle coupling body.

8. Syringe according to one or more of the preceding claims, characterized in that it comprises said elastic means arranged outside said cylindrical body and accommodated, in compressed position, below the button provided at the end of said plunger-like piston, said elastic means comprising a cylindrical spring which acts between the internal surface of said button and the flanged base of a collar arranged coaxially to said plunger-like piston, said collar being retained in position by tabs which end with an engagement tooth which can engage the lower face of said collar to keep said spring in compressed position, at the end of the injection stroke said engagement teeth engaging a truncated-cone abutment defined on said cylindrical body to open out said tabs and disengage said teeth from said collar.

9. Syringe according to one or more of the preceding claims, characterized in that it comprises a removable retainer rod which is connected to the button of said plunger-like piston and can abut against the flange defined at the end of the cylindrical body to prevent coupling between said plunger-like piston and said needle during the filling of the syringe with the liquid.
